# EUROPEAN PATENT SPECIFICATION

(11) **EP 4 051 307 B1**
(45) Date of publication and mention of the grant of the patent: **01.02.2023**
(21) Application number: 20771826.3
(22) Date of filing: 11.09.2020
(51) Int. Cl.: A61K 38/03, A61K 38/08, A61K 38/10, A61P 11/00, A61P 31/14

(54) **PEPTIDE FOR PREVENTION OR TREATMENT OF COVID-19**
PEPTID ZUR VORBEUGUNG ODER BEHANDLUNG VON COVID-19
PEPTIDE POUR LA PRÉVENTION OU LE TRAITEMENT DU COVID-19

(30) Priority: 08.05.2020 EP 20173713
(43) Date of publication of application: 07.09.2022
(73) Proprietor: APEPTICO Forschung und Entwicklung GmbH, 1150 Wien (AT)
(72) Inventor: FISCHER, Bernhard., 1160 Wien (AT)
(74) Representative: SONN Patentanwälte OG
(86) International application number: PCT/EP2020/075428
(87) International publication number: WO 2021/223894

(56) References cited:
- WO-A1-2012/065201
- WO-A2-2015/140125
- Lisa Vienna: "SOLNATIDE has been approved for the treatment of COVID-19 patients suffering from pulmonary oedema and acute respiratory distress syndrome by the Italian Medicines Agency and the Ethics Committee of the National Institute for Infectious Diseases", , 28 April 2020 (2020-04-28), XP055732424, Retrieved from the Internet: URL:https://www.lisavienna.at/news/solnati de-has-been-approved-for-the-treatment-of- covid-19-patients-suffering-from-pulmonary -oedema-a/ [retrieved on 2020-09-21]
- Biocom Ag: "COVID-19: Solnatide approved for compassionate use - European Biotechnology", , 8 April 2020 (2020-04-08), XP055732431, Retrieved from the Internet: URL:https://european-biotechnology.com/up- to-date/latest-news/news/covid-19-solnatid e-approved-for-compassionate-use.html [retrieved on 2020-09-21]
- Rtds-Verein Anonymous ET AL: "Exploration of safety, tolerability and clinical efficacy of solnatide IMP in patients infected with the 2019 new coronavirus Fact Sheet", , 1 April 2020 (2020-04-01), XP055732440, Retrieved from the Internet: URL:https://cordis.europa.eu/project/id/10 1003595/en?format=pdf [retrieved on 2020-09-21]
- QIQUAN ZHOU ET AL: "Solnatide Demonstrates Profound Therapeutic Activity in a Rat Model of Pulmonary Edema Induced by Acute Hypobaric Hypoxia and Exercise", CHEST, vol. 151, no. 3, 1 March 2017 (2017-03-01) , pages 658-667, XP055732446, US ISSN: 0012-3692, DOI: 10.1016/j.chest.2016.10.030
- Biocom Ag: "COVID-19: Apepticos Leitkandidat Solnatide erhält Zulassung - transkript", , 7 April 2020 (2020-04-07), XP055732448, Retrieved from the Internet: URL:https://transkript.de/news/covid-19-ap epticos-leitkandidat-solnatide-erhaelt-zul assung.html [retrieved on 2020-09-21]
- SUSAN J. TZOTZOS ET AL: "Incidence of ARDS and outcomes in hospitalized patients with COVID-19: a global literature survey", CRITICAL CARE, vol. 24, no. 1, 21 August 2020 (2020-08-21), XP055767224, DOI: 10.1186/s13054-020-03240-7
- Eunethta: "SOLNATIDE FOR THE TREATMENT OF COVID-19", , 1 August 2020 (2020-08-01), XP055767239, Retrieved from the Internet: URL:https://eunethta.eu/wp-content/uploads /2020/08/EUnetHTA-Covid-19_RCR06_SOLNATIDE _August2020_FINAL.pdf [retrieved on 2021-01-20]

## Description

The field of the present invention relates to peptides for prevention or treatment of Coronavirus disease 2019 (COVID-19).

COVID-19 is an infectious disease caused by severe acute respiratory syndrome coronavirus 2 (SARS-CoV-2). See for instance Rodriguez-Morales et al., 2020, PubMed unique identifier (PMID) 32179124, Jiang et al., 2020, PMID 32133578, and Tay et al., 2020, PMID 32346093. COVID-19 became pandemic in 2020.

The most common symptoms of COVID-19 are fever, dry cough, and tiredness. Other, less common symptoms include aches and pains, nasal congestion, headache, conjunctivitis, sore throat, diarrhoea, loss of taste or smell or a rash on skin or discoloration of fingers or toes.

Importantly, COVID-19 may affect the lungs causing pneumonia. In severe cases, COVID-19 may rapidly progress to acute respiratory distress syndrome (ARDS) causing respiratory failure, septic shock, or multi-organ failure. Further complications associated with COVID-19 include sepsis, abnormal clotting, and damage to the heart, kidneys, and liver.

Several pharmaceutical interventions have been suggested and are presently undergoing clinical trials: Among them are antiviral drugs such as remdesivir, favipiravir, lopinavir, ritonavir, ribavirin, taribavirin, umefenovir, amantadine, camostat and TMC-310911, chloroquines such as hydroxychloroquine and chloroquine, anti-interleukin-6 receptor antibodies such as sarilumab and tocilizumab, interferon-beta and angiotensin-converting enzyme 2. On 1 May 2020, the US-FDA issued an Emergency Use Authorization for remdesivir in patients hospitalized with severe COVID-19, after results of a large clinical trial had suggested it reduced the duration of recovery. Neither of these interventions have been shown to be effective in patients with severe and progressed COVID-19.

Furthermore, symptomatic treatments are known: Patients who are severely affected by COVID-19 may receive oxygen therapy, mechanical ventilation, renal replacement therapy (RRT) or extracorporeal membrane oxygenation (ECMO), typically in an intensive-care-unit (ICU) setting. According to the World Health Organisation (WHO), disease severity can be scored from 0-8 as shown in Table 1 below.

**Table 1 - WHO scoring scheme for COVID-19 severity**

| **Patient State** | **Descriptor** | **Score** |
|---|---|---|
| ***Uninfected*** | No clinical or virological evidence of infection | 0 |
| ***Ambulatory*** | No limitation of activities | 1 |
| | Limitation of activities | 2 |
| ***Hospitalized Mild disease*** | Hospitalized, no oxygen therapy | 3 |
| | Oxygen by mask or nasal prongs | 4 |
| ***Hospitalized Severe Disease*** | Non-invasive ventilation or high-flow oxygen | 5 |
| | Intubation and mechanical ventilation | 6 |
| | Ventilation + additional organ support - pressors, RRT, ECMO | 7 |
| ***Dead*** | Death | 8 |

However, there remains a desperate need for safe and effective pharmaceutical interventions against COVID-19. It is therefore an object of the present invention to provide a pharmaceutical prevention or treatment of COVID-19, in particular severe COVID-19 (e.g. with a severity score above 4).

The present invention relates to a peptide, wherein the peptide consists of 7-17 amino acids and includes the hexamer TX₁EX₂X₃E (SEQ ID NO: 6), wherein X₁, X₂ and X₃ can be any natural or non-natural amino acid, and wherein the peptide does not exhibit TNF-receptor-binding activity, for use in prevention or treatment of COVID-19 in a patient. Such peptides are also called TIP peptides herein.

The present invention also provides a method for delaying the onset of or treating COVID-19, comprising
- obtaining a pharmaceutically acceptable formulation comprising a peptide, wherein the peptide consists of 7-17 amino acids and includes the hexamer TX₁EX₂X₃E (SEQ ID NO: 6), wherein X₁, X₂ and X₃ can be any natural or non-natural amino acid, and wherein the peptide does not exhibit TNF-receptor-binding activity; and
- administering an effective amount of the formulation to a patient having COVID-19 or at risk of developing COVID-19;
preferably wherein the peptide is further defined or for use as set forth herein.

A TIP peptide, namely AP301 or solnatide (cyclo-CGQRETPEGAEAKPWYC; CGQRETPEGAEAKPWYC is SEQ ID NO: 1), has received a compassionate use authorization for treatment of COVID-19 patients in Austria and Italy. A clinical trial is presently ongoing (see example section below).

TIP peptides are peptides comprising the human tumour necrosis factor (TNF) lectin-like domain (TIP domain). The TIP domain is covered for instance by van der Goot et al, 1999, PMID 10571070. As used herein, TIP peptides consist of 7-17 amino acids including the hexamer TX₁EX₂X₃E (SEQ ID NO: 6), wherein X₁, X₂ and X₃ can be any natural or non-natural amino acid, wherein the peptide does not exhibit TNF-specific inflammatory activity (Hribar et al, 1999, PMID 10540321; Elia et al, 2003, PMID 12842853) and may be cyclised. The biological activity of TIP peptides such as AP301 or solnatide (cyclo-CGQRETPEGAEAKPWYC; CGQRETPEGAEAKPWYC is SEQ ID NO: 1) comprises activation of the amiloride-sensitive epithelial sodium channel (ENaC), as reported by Tzotzos et al, 2013, PMID 23313096.

Unrelated to COVID-19, TIP peptides are known for instance from European Patents EP 1 247 531 B1 and EP 1 264 599 B1 for use in the treatment of oedema, especially pulmonary oedema. Such peptides are also known for use in the treatment and prevention of vascular complications in diabetes patients such as micro- and macroangiopathy, myocardial infarction, microvascular cardiac hyperpermeability, stroke, neuropathy, retinopathy, nephropathy or diabetic foot disease (EP 2 582 385 B1). Moreover, such peptides are known for prevention of oedema due to reduction in hyperpermeability, caused by injury of endothelial and/or epithelial layers (EP 2 403 519 B1, also published as WO 2010/099556 A1). In addition, such peptides are known for use in the treatment and prevention of the pulmonary form of altitude sickness (WO 2014/001177 A1). Such peptides are also known for use in treatment or prevention of influenza, when administered together with an inhibitor of viral neuraminidase (WO 2012/065201 A1). Finally, WO 2015/140125 A2 relates to dry-powder formulations of TIP peptides.

The peptide for use in the present invention is particularly suitable for a patient before, after or during oxygen therapy or ventilation. Accordingly, in a preferred embodiment, the patient has received or is receiving or is at risk of receiving an oxygen therapy (e.g. by mask or nasal prongs). In another preferred embodiment, the patient has thus been ventilated or is being ventilated or is at risk of being ventilated, such as by non-invasive ventilation, preferably non-invasive positive pressure ventilation, or by mechanical ventilation.

In a particularly preferred embodiment, the peptide is for use in a patient who is in a hospital, preferably in an ICU, or is at risk of being admitted to a hospital, preferably an ICU, for management of COVID-19.

To further increase its beneficial effect in the lungs, the peptide is preferably administered to the patient by inhalation, preferably by oral inhalation or by endotracheal inhalation.

The peptide may be administered (in a pharmaceutical composition) via a dry-powder inhaler. Examples of such powder inhalers, which may be employed in the present invention, are described in US Patent Nos. 4,995,385 and 4,069,819; already established products are SPINHALER^{®}, ROTAHALER^{®}, FLOWCAPS^{®}, INHALATOR^{®}, DISKHALER^{®} and AEROLIZER^{®}. A particularly preferred dry-powder formulation of the peptide is disclosed in WO 2015/140125 A2.

Alternatively, the peptide may be administered (in a pharmaceutical composition) via a nebuliser, typically continuously as long as the nebuliser is switched on or breathactuated. Examples of such nebulisers are established products such as Aeroneb^{®} and Pari^{®}. Document US 9,364,618 B2, e.g., also discloses a nebuliser.

Finally, the peptide (in a pharmaceutical composition) may be administered via a metered-dose inhaler. A metered-dose inhaler is a device that aerosolises a pre-defined dose of a pharmaceutical composition (i.e. produces a comparatively short burst of aerosol with a defined dose), usually for selfadministration by the patient. A metered-dose inhaler is for instance disclosed in US 6,260,549 B1.

Of course, the peptide may also be administered in other manners, e.g. intravenously.

According to a particular preference, the peptide is administered to the patient at a daily dose of 10 mg to 500 mg, preferably 50 mg to 400 mg, more preferably 75 mg to 350 mg, even more preferably 100 mg to 300 mg, yet even more preferably 125 mg to 175 mg, especially 150 mg to 250 mg or even 175 mg to 225 mg.

To further increase effectivity, the peptide is preferably administered to the patient for at least two days, preferably at least three days, even more preferably at least four days, yet even more preferably at least five days, especially at least six days or even at least seven days.

The peptide may be administered together with other medicaments or alone. In a preferred embodiment, the patient additionally has undergone, is undergoing or will be undergoing a therapy with at least one compound selected from the group consisting of antiviral drugs such as remdesivir, favipiravir, lopinavir, ritonavir, ribavirin, taribavirin, umefenovir, amantadine, camostat and TMC-310911, chloroquines such as hydroxychloroquine and chloroquine, anti interleukin-6 receptor antibodies such as sarilumab and tocilizumab, interferon-beta and angiotensin-converting enzyme 2.

The peptide disclosed herein is also suitable for prevention, especially in patients at high risk of SARS-CoV-2 infection, such as medical personnel treating COVID-19 patients. Therefore, in a preferred embodiment of the present invention, the patient is at risk of developing COVID-19, preferably wherein the patient is medical personnel and/or wherein the patient has been, is or is likely to be in close contact with another individual infected with SARS-CoV-2. In particular for such a patient, administration of the peptide via dry-powder inhaler or metered-dose inhaler is particularly suitable. Herein, "close contact" is defined as a contact with a minimum duration of 10 minutes within a proximity of 2 meters.

In a further preferred embodiment, the inventive peptide is for reducing the risk of the patient deteriorating to COVID-19 with a severity score of 5 or 6 according to the WHO scoring scheme (see Table 1 above), preferably wherein the patient has COVID-19 with a severity score of 4 according to the WHO scoring scheme, or for reducing the risk of the patient deteriorating to COVID-19 with a severity score of 7 or 8 according to the WHO scoring scheme, preferably wherein the patient has COVID-19 with a severity score of 6 according to the WHO scoring scheme.

In yet another preferred embodiment, the inventive peptide is for reducing the severity score of COVID-19 according to the WHO scoring scheme (see Table 1 above) in the patient, in particular from 6 or 7 to 4 or 5.

The sequential (or sepsis) organ failure assessment score (SOFA score) can be used to track a patient's status during the stay in an ICU to determine the extent of the patient's organ function or rate of failure. The score is based on six different scores, one each for the respiratory, cardiovascular, hepatic, coagulation, renal and neurological systems (see Vincent, J-L., et al. "The SOFA (Sepsis-related Organ Failure Assessment) score to describe organ dysfunction/failure." (1996): 707-710., PMID 8844239). The lower the SOFA score, the number of life-threatening organ failures decreases, and the patient has a higher probability for survival. The present invention turned out to be highly suitable for reducing the SOFA score in COVID-19 patients (see example 2). Therefore, in a preference, the patient (to be treated) has a SOFA score of at least 8, preferably at least 9, more preferably at least 10, even more preferably at least 11, especially at least 12. In a further preferred embodiment, the present invention is for increasing the organ function of the patient or for decreasing the SOFA score of the patient.

Lung compliance is a measure of the lung's ability to stretch and expand (distensibility of elastic tissue). Low compliance indicates a stiff lung, whereas a higher compliance demonstrates a more healthy and elastic lung. The present invention turned out to be highly suitable for increasing lung compliance in COVID-19 patients (see example 2). Therefore, in a preference, the patient (to be treated) has a dynamic lung compliance (also called C_{dyn}, and measured in mL / cm H₂O) of at most 50, preferably at most 45, more preferably at most 40, even more preferably at most 35. In a further preferred embodiment, the present invention is for increasing lung compliance, in particular dynamic lung compliance, of the patient.

Normal human body-temperature (normothermia, euthermia) is the typical temperature range found in humans. The normal human body temperature range is typically stated as 36.5-37.5 °C. The body temperature is a common measure for the overall status of a patient. The present invention turned out to be highly suitable for decreasing body temperature in COVID-19 patients (see example 2). Therefore, in a preference, the patient (to be treated) has a body temperature of more than 37.5°C, especially more than 38°C. In a further preferred embodiment, the present invention is for reducing the body temperature of the patient.

In also turned out that the present invention is especially suitable for reducing risk of mortality (see example 2). Accordingly, in a further preferred embodiment, the present invention is for reducing risk of mortality of the patient.

Peptides for use according to the present invention are known per se, e.g. from the following patent documents: EP 1 264 599 B1, US 2007/299003 A1, WO 94/18325 A1, WO 00/09149 A1, WO 2006/013183 A1 and WO 2008/148545 A1.

In a preference, the peptide for use according to the invention includes the amino-acid hexamer TPEGAE (SEQ ID NO: 2), which is the potential carbohydrate-binding motif (Marquardt et al, 2007, PMID 17918767) and may increase biological activity.

In a preferred embodiment of the present invention, the peptide for use according to the present invention is cyclic (or circularised), in order to retain the original TNF-alpha conformation as much as possible (Elia et al, 2003, PMID 12842853), which may lead to higher biological activity. This is an optional feature, because, according to Marquard et al, 2007, PMID 17918767, peptide cyclisation may not be essential for carbohydrate binding important for biological activity.

In a preferred embodiment, the peptide of the present invention includes the amino-acid hexamer TPEGAE (SEQ ID NO: 2). Preferably, the peptide is cyclic and contains a sequence of consecutive amino acids selected from the group comprising
- QRETPEGAEAKPWY (SEQ ID NO: 3)
- PKDTPEGAELKPWY (SEQ ID NO: 4)
- CGQRETPEGAEAKPWYC (SEQ ID NO: 1)
- CGPKDTPEGAELKPWYC (SEQ ID NO: 5) and
- fragments of at least seven amino acids containing the hexamer TPEGAE (SEQ ID NO: 2). Such peptides or fragments thereof can have a biological activity as for instance shown in WO 2014/001177.

In another preferred embodiment of the invention, the peptide is administered as a single active agent.

In a particularly preferred embodiment, the peptide according to the present invention is the peptide AP301 or solnatide (CGQRETPEGAEAKPWYC, SEQ ID NO: 1), wherein solnatide is cyclised via the C residues, preferably by a disulfide bond between the C residues.

In preferred embodiments, the peptide for use in the present invention may be formulated in a pharmaceutical composition. The expression "pharmaceutical composition" refers to any composition comprising at least one active agent (i.e. the peptide), and preferably one or more excipients, which is pharmaceutically acceptable for administration (in particular for administration by inhalation) to an individual, especially a mammal, in particular a human. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives, such as benzalkonium chloride. The pharmaceutical composition according to the present invention may be liquid or ready to be dissolved in liquid such as sterile, deionised or distilled water, or sterile isotonic phosphate-buffered saline (PBS). Preferably, 1000 µg (dryweight) of such a composition comprises or consists of 0.1-990 pg, preferably 1-900pg, more preferably 10-200µg of the peptide, and optionally 1-500 pg, preferably 1-100 pg, more preferably 5-15 µg (buffer) salts (preferably to yield an isotonic buffer in the final volume), and optionally 0.1-999.9 pg, preferably 100-999.9 pg, more preferably 200-999 µg other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml. The dosage and method of administration, however, typically depends on the individual to be treated. In general, the peptide may be administered at a dose of between 1 pg/kg and 10 mg/kg, more preferably between 10 pg/kg and 5 mg/kg, most preferably between 0.1 and 2 mg/kg.

As used herein, a peptide according to the present invention having "no TNF-receptor-binding activity" or "not exhibiting TNF-receptor-binding activity" shall mean: said peptide having/exhibiting no TNF-receptor-binding activity that is sufficient to cause TNF-specific inflammatory activity detrimental to the successful treatment of a patient.

In particular, "TNF-specific inflammatory activity detrimental to the successful treatment of a patient" may mean the following: In an *ex vivo* safety pharmacological study of said peptide in a human whole blood sample - performed to assess whether addition of said peptide results in the release of the pro-inflammatory marker interleukin-6 (IL-6) from fresh human whole blood - addition of said peptide up to a concentration of 10mg/ml to said blood sample results in less than 0.5 pg/ml released IL-6 (cf. EP 2 582 385 A1, example 2).

Herein, the term "efficient amount" (of the peptide) is the amount sufficiently efficient to cause the intended therapeutic or prophylactic effect, for instance to subdue a further worsening of the disease or condition, treat such worsening or foster curing or cure the disease or condition. Usually, the efficient amount is formulated for an average patient. However, the actually efficient amounts (or doses) may be formulated as to depend on one or more selected from group comprising: particular mode of administration; age, body weight, general health of the patient; severity and progression of the disease or condition.

The invention is further described in the following examples and figures, yet without being restricted thereto.
Fig. 1 shows the mean sequential organ failure assessment (SOFA) score of COVID-19 patients treated twice daily with solnatide over a period of 7 days.
Fig. 2 shows the mean dynamic lung compliance values (in mL / cm H₂O) of COVID-19 patients treated twice daily with solnatide over a period of 7 days.
Fig. 3 shows the mean body temperature (in °C) of COVID-19 patients treated twice daily with solnatide over a period of 7 days.

### Example 1 - Clinical trial in COVID-19 patients

On 11 April 2020, the "COV-2-SOLNATIDE-20" clinical trial of solnatide was started in mechanically ventilated COVID-19 patients (European Union Drug Regulating Authorities Clinical Trials Database - EudraCT number 2020-001244-26).

This is a phase II, randomized, placebo-controlled, double-blinded pilot study. Treatment duration for each patient is for up to 7 days and study duration is for up to 28 days. About 40 patients are randomized into either the placebo or 200 mg solnatide per day group in a 1:1 ratio.

Administration is by endotracheal inhalation via Aeroneb^{®} nebuliser. Solnatide is formulated as powder for reconstitution, and is reconstituted in water for injection.

Solnatide group: 14 pulmonary administered doses of 100 mg solnatide, each reconstituted double-blinded in water for injection, every 12 hours. Placebo group: 14 pulmonary administered doses of placebo (i.e. water for injection), every 12 hours.

Endpoints include: Days free of mechanical ventilation (ventilator free days, VFD) within 28 days, time to extubation through day 28, all-cause deaths through day 28, score change according to the WHO scoring scheme of Table 1 (assessed at day 14 and 28 after randomization), days of hospitalization through day 28 and ICU days through day 28.

It is very plausible that the peptide of the present invention leads to improvement with regard to at least one of these endpoints.

### Example 2

Community patients with serious health conditions were admitted to a local hospital for examination. In some of these patients, COVID-19 was confirmed by positive testing for SARS-CoV-2 by nasopharyngeal swab and real-time RT-PCR.

Following worsening of conditions, seriously ill COVID-19 patients were admitted to the ICU of the hospital. Half of patients were treated with orally inhaled solnatide for 7 days twice daily, in addition to standard critical care treatment and life support.

In parallel, another cohort of serious COVID-19 patients with comparable life-threatening conditions were treated at ICU of the hospital. These patients were treated with placebo only in addition to standard critical care treatment and life support.

During an observation period of 7 days, patient-centred clinical parameters which reflect the severity of COVID-19 were recorded.

During an observation period of 7 days, patient-centred clinical parameters which reflect the severity of COVID-19 were recorded.

The SOFA score was used to track a patient's status during the stay in an intensive care unit (ICU) to determine the extent of the patient's organ function or rate of failure.

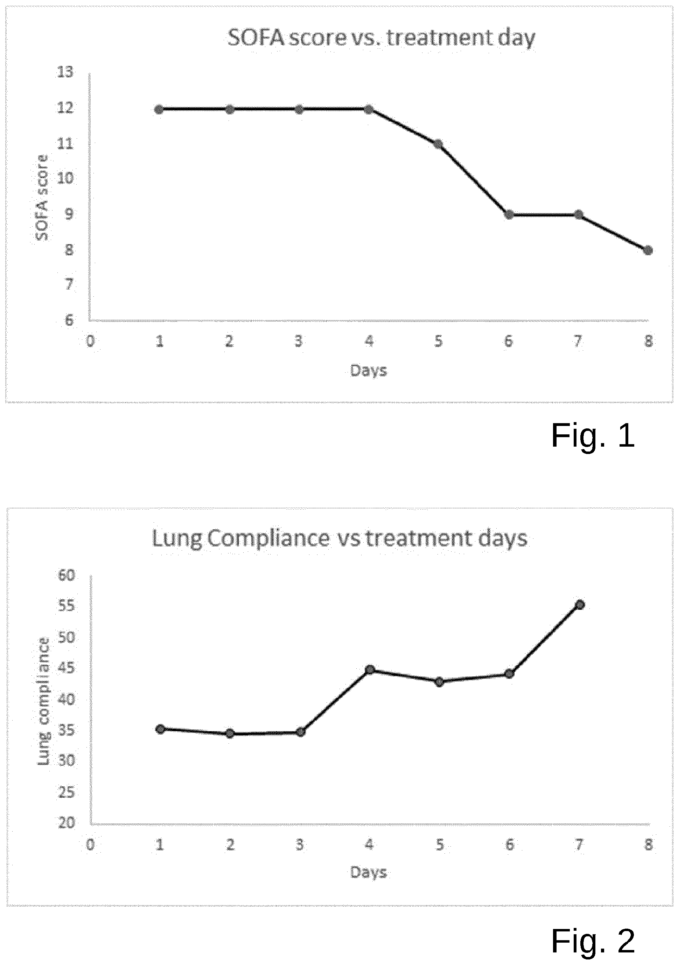

Fig. 1 shows the mean SOFA score of COVID-19 patients treated twice daily with solnatide over a period of 7 days. The mean SOFA score decreased from 12 to 8 over the treatment period.

By contrast, SOFA score of severe COVID-19 patients without solnatide treatment did not decrease in the same interval.

Fig. 2 shows the mean dynamic lung compliance values of COVID-19 patients treated twice daily with solnatide over a period of 7 days. The mean dynamic lung compliance increased from approx. 35 mL / cm H₂O to 50 mL / cm H₂O over the treatment period.

By contrast, dynamic lung compliance of severe COVID-19 patients without solnatide treatment remained below 40 mL / cm H₂O in the same interval.

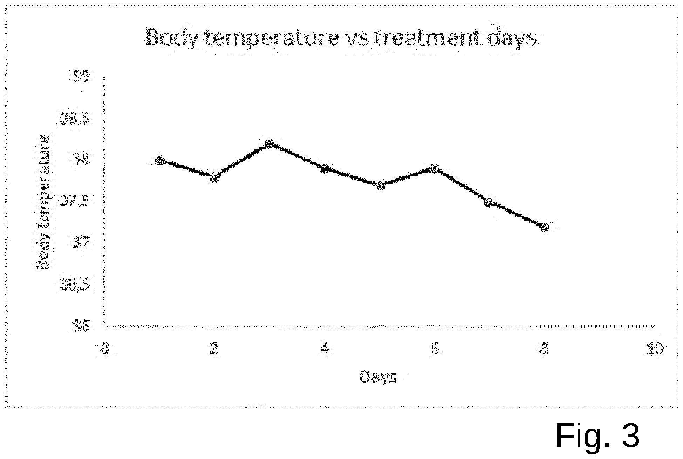

Fig. 3 shows the mean body temperature of COVID-19 patients treated twice daily with solnatide over a period of 7 days. The mean body temperature was elevated at the initiation of the treatment, but decreased during the treatment period of 7 days.

By contrast, the body temperature of severe COVID-19 patients without solnatide treatment remained above 38°C all the time.

It is known from the scientific literature that mortality of severe COVID-19 patients is as high as 50% of patients treated in the ICU (Tzotzos et al. Incidence of ARDS and outcomes in hospitalized patients with COVID-19. Critical Care (2020) 24:516; PMID 32825837). In the present observation, none of the severe COVID-19 patients treated with solnatide died within 28 days after onset of COVID-19 symptoms. By contrast, in the comparator cohort composed of equally severe COVID-19 patients, 3 patients died within 28 days.

## Claims

1. A peptide, wherein the peptide consists of 7-17 amino acids and includes the hexamer TX₁EX₂X₃E, wherein X₁, X₂ and X₃ can be any natural or non-natural amino acid, and wherein the peptide does not exhibit TNF-receptor-binding activity, for use in prevention or treatment of COVID-19 in a patient.

2. The peptide for use according to claim 1, wherein the patient has received or is receiving or is at risk of receiving an oxygen therapy.

3. The peptide for use according to claim 1 or 2, wherein the patient has been ventilated or is being ventilated or is at risk of being ventilated, such as by non-invasive ventilation, preferably non-invasive positive pressure ventilation, or by mechanical ventilation; and/or wherein the patient is in a hospital, preferably in an intensive care unit (ICU), or is at risk of being admitted to a hospital, preferably an ICU, for management of COVID-19.

4. The peptide for use according to any one of claims 1 to 3, wherein the peptide is administered to the patient by inhalation, preferably by oral inhalation or by endotracheal inhalation.

5. The peptide for use according to any one of claims 1 to 4, wherein the peptide is administered to the patient at a daily dose of 10 mg to 500 mg, preferably 50 mg to 400 mg, more preferably 75 mg to 350 mg, even more preferably 100 mg to 300 mg, yet even more preferably 125 mg to 175 mg, especially 150 mg to 250 mg or even 175 mg to 225 mg; and/or
wherein the peptide is administered to the patient for at least two days, preferably at least three days, even more preferably at least four days, yet even more preferably at least five days, especially at least six days or even at least seven days.

6. The peptide for use according to any one of claims 1 to 5, wherein the patient additionally has undergone, is undergoing or will be undergoing a therapy with at least one compound selected from the group consisting of antiviral drugs such as remdesivir, favipiravir, lopinavir, ritonavir, ribavirin, taribavirin, umefenovir, amantadine, camostat and TMC-310911, chloroquines such as hydroxychloroquine and chloroquine, anti interleukin-6 receptor antibodies such as sarilumab and tocilizumab, interferon-beta and angiotensin-converting enzyme 2.

7. The peptide for use according to any one of claims 1 to 6, wherein the patient is at risk of developing COVID-19, preferably wherein the patient is medical personnel and/or wherein the patient has been, is or is likely to be in close contact with another individual infected with SARS-CoV-2.

8. The peptide for use according to any one of claims 1 to 6, for reducing the risk of the patient deteriorating to COVID-19 with a severity score of 5 or 6 according to the WHO scoring scheme, preferably wherein the patient has COVID-19 with a severity score of 4 according to the WHO scoring scheme, or for reducing the risk of the patient deteriorating to COVID-19 with a severity score of 7 or 8 according to the WHO scoring scheme, preferably wherein the patient has COVID-19 with a severity score of 6 according to the WHO scoring scheme; or for reducing the severity score of COVID-19 according to the WHO scoring scheme in the patient, in particular from 6 or 7 to 4 or 5.

9. The peptide for use according to any one of claims 1 to 8, wherein the peptide includes the amino-acid hexamer TPEGAE.

10. The peptide for use according to any one of claims 1 to 9, wherein the peptide is cyclic; preferably wherein the peptide contains a sequence of consecutive amino acids selected from the group consisting of
- QRETPEGAEAKPWY
- PKDTPEGAELKPWY
- CGQRETPEGAEAKPWYC
- CGPKDTPEGAELKPWYC and
- fragments thereof having a length of at least seven amino acids and containing the hexamer TPEGAE.

11. The peptide for use according to any one of claims 1 to 10, wherein the peptide consists of the amino acid sequence CGQRETPEGAEAKPWYC and is preferably cyclised, preferably via the C residues, especially by a disulfide bond between the C residues.

12. The peptide for use according to any one of claims 1 to 11, wherein the patient has a sequential organ failure assessment (SOFA) score of at least 8, preferably at least 9, more preferably at least 10, even more preferably at least 11, especially at least 12; and/or wherein the peptide is for increasing the organ function of the patient or for decreasing the SOFA score of the patient.

13. The peptide for use according to any one of claims 1 to 12, wherein the patient has a lung compliance of at most 50, preferably at most 45, more preferably at most 40, even more preferably at most 35 mL / cm H₂O; and/or wherein the peptide is for increasing lung compliance, in particular dynamic lung compliance, of the patient.

14. The peptide for use according to any one of claims 1 to 13, wherein the patient has a body temperature of more than 37.5°C, especially more than 38°C; and/or wherein the peptide is for reducing the body temperature of the patient.

15. The peptide for use according to any one of claims 1 to 14, for reducing risk of mortality of the patient.

## Patentansprüche

1. Peptid, wobei das Peptid aus 7-17 Aminosäuren besteht und das Hexamer TX₁EX₂X₃E einschließt, wobei X₂, X₂ und X₃ jede natürliche oder nicht-natürliche Aminosäure sein kann, und wobei das Peptid keine TNF-Rezeptor-Bindungsaktivität zeigt, zur Anwendung bei der Vorbeugung oder Behandlung von COVID-19 bei einem Patienten.

2. Peptid zur Anwendung nach Anspruch 1, wobei der Patient eine Sauerstofftherapie erhalten hat oder erhält oder bei dem das Risiko besteht, eine zu erhalten.

3. Peptid zur Anwendung nach Anspruch 1 oder 2, wobei der Patient beatmet wurde oder beatmet wird oder bei dem das Risiko besteht, beatmet zu werden, wie mit nicht-invasiver Beatmung, vorzugsweise nicht-invasiver Überdruckbeatmung, oder mit mechanischer Beatmung; und/oder wobei sich der Patient in einem Krankenhaus befindet, vorzugsweise auf einer Intensivstation (ITS), oder das Risiko besteht, dass er zur Behandlung von COVID-19 in einem Krankenhaus, vorzugsweise auf einer Intensivstation, aufgenommen wird.

4. Peptid zur Anwendung nach einem der Ansprüche 1 bis 3, wobei das Peptid dem Patienten durch Inhalation, vorzugsweise durch orale Inhalation oder durch endotracheale Inhalation, verabreicht wird.

5. Peptid zur Anwendung nach einem der Ansprüche 1 bis 4, wobei dem Patienten das Peptid in einer täglichen Dosis von 10 mg bis 500 mg, vorzugsweise 50 mg bis 400 mg, bevorzugter 75 mg bis 350 mg, noch bevorzugter 100 mg bis 300 mg, noch bevorzugter 125 mg bis 175 mg, insbesondere 150 mg bis 250 mg oder sogar 175 mg bis 225 mg verabreicht wird; und/oder
wobei dem Patienten das Peptid für mindestens zwei Tage, vorzugsweise mindestens drei Tage, noch bevorzugter mindestens vier Tage, noch bevorzugter mindestens fünf Tage, insbesondere mindestens sechs Tage oder sogar mindestens sieben Tage verabreicht wird.

6. Peptid zur Anwendung nach einem der Ansprüche 1 bis 5, wobei sich der Patient zusätzlich einer Therapie mit mindestens einer Verbindung ausgewählt aus der Gruppe, bestehend aus Virostatika, wie Remdesivir, Favipiravir, Lopinavir, Ritonavir, Ribavirin, Taribavirin, Umefenovir, Amantadin, Camostat und TMC-310911, Chloroquinen, wie Hydroxychloroquin und Chloroquin, Anti-Interleukin-6-Rezeptor-Antikörpern, wie Sarilumab und Tocilizumab, Interferon-beta und Angiotensin-konverterierendem Enzym 2, unterzogen hat, sich unterzieht oder sich unterziehen wird.

7. Peptid zur Anwendung nach einem der Ansprüche 1 bis 6, wobei bei dem Patienten das Risiko besteht, COVID-19 zu entwickeln, wobei der Patient vorzugsweise medizinisches Personal ist und/oder wobei der Patient in engem Kontakt mit einem anderen mit SARS-CoV-2 infizierten Individuum stand, steht oder voraussichtlich stehen wird.

8. Peptid zur Anwendung nach einem der Ansprüche 1 bis 6, zur Verringerung des Risikos, dass der Patient COVID-19 mit einem Schweregrad-Score von 5 oder 6 gemäß der WHO-Schweregradeinteilung entwickelt, wobei der Patient vorzugsweise COVID-19 mit einem Schweregrad-Score von 4 gemäß der WHO-Schweregradeinteilung hat, oder zur Verringerung des Risikos, dass der Patient COVID-19 mit einem Schweregrad-Score von 7 oder 8 gemäß der WHO-Schwergradeinteilung entwickelt, wobei der Patient COVID-19 mit einem Schweregrad-Score von 6 gemäß der WHO-Schweregradeinteilung hat; oder zur Verringerung des Schweregrad-Scores von COVID-19 gemäß der WHO-Schwergradeinteilung bei dem Patienten, insbesondere von 6 oder 7 auf 4 oder 5.

9. Peptid zur Anwendung nach einem der Ansprüche 1 bis 8, wobei das Peptid das Aminosäure-Hexamer TPEGAE einschließt.

10. Peptid zur Anwendung nach einem der Ansprüche 1 bis 9, wobei das Peptid zyklisch ist; wobei das Peptid vorzugsweise eine Sequenz von aufeinanderfolgenden Aminosäuren enthält, ausgewählt aus der Gruppe, bestehend aus
- QRETPEGAEAKPWY
- PKDTPEGAELKPWY
- CGQRETPEGAEAKPWYC
- CGPKDTPEGAELKPWYC und
- Fragmenten davon, die eine Länge von mindestens sieben Aminosäuren haben und das Hexamer TPEGAE enthalten.

11. Peptid zur Anwendung nach einem der Ansprüche 1 bis 10, wobei das Peptid aus der Aminosäuresequenz CGQRETPEGAEAKPWYC besteht und vorzugsweise zyklisiert ist, vorzugsweise über die C-Reste, insbesondere durch eine Disulfidbindung zwischen den C-Resten.

12. Peptid zur Anwendung nach einem der Ansprüche 1 bis 11, wobei der Patient einen Score zur Bewertung von sequenziellem Organversagen (SOFA) von mindestens 8, vorzugsweise mindestens 9, noch bevorzugter mindestens 10, noch bevorzugter mindestens 11, insbesondere mindestens 12 hat; und/oder wobei das Peptid zur Erhöhung der Organfunktion des Patienten oder zur Verringerung des SOFA-Scores des Patienten dient.

13. Peptid zur Anwendung nach einem der Ansprüche 1 bis 12, wobei der Patient eine Lungencompliance von höchstens 50, vorzugsweise höchstens 45, noch bevorzugter höchstens 40, noch bevorzugter höchstens 35 ml/cmH₂O aufweist; und/oder wobei das Peptid zur Erhöhung der Lungencompliance, insbesondere der dynamischen Lungencompliance, des Patienten dient.

14. Peptid zur Anwendung nach einem der Ansprüche 1 bis 13, wobei der Patient eine Körpertemperatur von mehr als 37,5 °C, insbesondere mehr als 38 °C, hat; und/oder wobei das Peptid zur Verringerung der Körpertemperatur des Patienten dient.

15. Peptid zur Anwendung nach einem der Ansprüche 1 bis 14 zur Verringerung des Sterblichkeitsrisikos des Patienten.

## Revendications

1. Peptide, où le peptide consiste en 7 à 17 acides aminés et inclut l'hexamère TX₁EX₂X₃E, où X₁, X₂ et X₃ peuvent être tout acide aminé naturel ou non naturel, et où le peptide ne présente pas d'activité de liaison au récepteur du TNF, destiné à être utilisé dans la prévention ou le traitement de la COVID-19 chez un patient.

2. Peptide pour utilisation selon la revendication 1, où le patient a reçu ou reçoit ou est à risque de recevoir une oxygénothérapie.

3. Peptide pour utilisation selon la revendication 1 ou 2, où le patient a été ventilé ou est ventilé ou est à risque d'être ventilé, par exemple par ventilation non invasive, de préférence ventilation à pression positive non invasive, ou par ventilation mécanique; et/ou où le patient est dans un hôpital, de préférence dans une unité de soins intensifs (USI), ou est à risque d'être admis dans un hôpital, de préférence une USI, pour le traitement de la COVID-19.

4. Peptide pour utilisation selon l'une quelconque des revendications 1 à 3, où le peptide est administré au patient par inhalation, de préférence par inhalation orale ou par inhalation endotrachéale.

5. Peptide pour utilisation selon l'une quelconque des revendications 1 à 4,
où le peptide est administré au patient à une dose quotidienne de 10 mg à 500 mg, de préférence 50 mg à 400 mg, de préférence encore 75 mg à 350 mg, de préférence encore 100 mg à 300 mg, de préférence encore 125 mg à 175 mg, en particulier 150 mg à 250 mg ou même 175 mg à 225 mg; et/ou
où le peptide est administré au patient pendant au moins deux jours, de préférence au moins trois jours, de préférence encore au moins quatre jours, de préférence encore au moins cinq jours, en particulier au moins six jours ou même au moins sept jours.

6. Peptide pour utilisation selon l'une quelconque des revendications 1 à 5, où le patient a en outre subi, subit ou subira une thérapie avec au moins un composé choisi dans le groupe consistant en les médicaments antiviraux tels que le remdesivir, le favipiravir, le lopinavir, le ritonavir, la ribavirine, la taribavirine, l'umefénovir, l'amantadine, le camostat et TMC-310911, les chloroquines telles que l'hydroxychloroquine et la chloroquine, les anticorps anti-récepteur de l'interleukine-6 tels que le sarilumab et le tocilizumab, l'interféron bêta et l'enzyme de conversion de l'angiotensine 2.

7. Peptide pour utilisation selon l'une quelconque des revendications 1 à 6, où le patient est à risque de développer la COVID-19, de préférence où le patient est du personnel médical et/ou où le patient a été, est ou est susceptible d'être en contact étroit avec un autre individu infecté par le SARS-CoV-2.

8. Peptide pour utilisation selon l'une quelconque des revendications 1 à 6, pour réduire le risque de détérioration du patient en COVID-19 avec un score de gravité de 5 ou 6 selon le schéma de notation de l'OMS, de préférence où le patient a la COVID-19 avec un score de gravité de 4 selon le schéma de notation de l'OMS, ou pour réduire le risque de détérioration du patient en COVID-19 avec un score de gravité de 7 ou 8 selon le schéma de notation de l'OMS, de préférence où le patient a la COVID-19 avec un score de gravité de 6 selon le schéma de notation de l'OMS; ou pour réduire le score de gravité de la COVID-19 selon le schéma de notation de l'OMS chez le patient, en particulier de 6 ou 7 à 4 ou 5.

9. Peptide pour utilisation selon l'une quelconque des revendications 1 à 8, où le peptide inclut l'hexamère d'acides aminés TPEGAE.

10. Peptide pour utilisation selon l'une quelconque des revendications 1 à 9, où le peptide est cyclique; de préférence où le peptide contient une séquence d'acides aminés consécutifs choisie dans le groupe consistant en
- QRETPEGAEAKPWY
- PKDTPEGAELKPWY
- CGQRETPEGAEAKPWYC
- CGPKDTPEGAELKPWYC et
- les fragments de celle-ci ayant une longueur d'au moins sept acides aminés et contenant l'hexamère TPEGAE.

11. Peptide pour utilisation selon l'une quelconque des revendications 1 à 10, où le peptide consiste en la séquence d'acides aminés CGQRETPEGAEAKPWYC et est de préférence cyclisé, de préférence par l'intermédiaire des résidus C, en particulier par une liaison disulfure entre les résidus C.

12. Peptide pour utilisation selon l'une quelconque des revendications 1 à 11, où le patient a un score d'évaluation de défaillance d'organe séquentielle (SOFA) d'au moins 8, de préférence d'au moins 9, de préférence encore d'au moins 10, de préférence encore d'au moins 11, en particulier d'au moins 12; et/ou où le peptide est pour augmenter la fonction d'organe du patient ou pour diminuer le score SOFA du patient.

13. Peptide pour utilisation selon l'une quelconque des revendications 1 à 12, où le patient a une compliance pulmonaire d'au plus 50, de préférence d'au plus 45, de préférence encore d'au plus 40, de préférence encore d'au plus 35 mL/cm H₂O; et/ou où le peptide est pour augmenter la compliance pulmonaire, en particulier la compliance pulmonaire dynamique, du patient.

14. Peptide pour utilisation selon l'une quelconque des revendications 1 à 13, où le patient a une température corporelle de plus de 37,5°C, en particulier de plus de 38°C; et/ou où le peptide est pour réduire la température corporelle du patient.

15. Peptide pour utilisation selon l'une quelconque des revendications 1 à 14, pour réduire le risque de mortalité du patient.
